# EUROPEAN PATENT APPLICATION

(11) **EP 4 628 578 A1**
(43) Date of publication of application: **08.10.2025**
(21) Application number: 23896875.4
(22) Date of filing: 30.11.2023
(51) Int. Cl.: C12N 7/02

(54) **METHOD FOR SEPARATING AND PURIFYING CVB1**

(30) Priority: 01.12.2022 CN 202211541435
(71) Applicant: Hangzhou Yangshengtang Biopharma Co., Ltd., Hangzhou, Zhejiang 310024 (CN)
(72) Inventor: YANG, Lisheng, Hangzhou, Zhejiang 310024 (CN); LIU, Bing, Hangzhou, Zhejiang 310024 (CN); DING, Tianyu, Hangzhou, Zhejiang 310024 (CN); JI, Chengfa, Hangzhou, Zhejiang 310024 (CN); WANG, Limin, Hangzhou, Zhejiang 310024 (CN); SHI, Wei, Hangzhou, Zhejiang 310024 (CN)
(74) Representative: J A Kemp LLP
(86) International application number: PCT/CN2023/135448
(87) International publication number: WO 2024/114733

(57) **Abstract**

The present invention relates to a method for separating and purifying CVB1, and particularly, relates to a method for purifying CVB1 virus by using a heparin affinity medium.

## Description

The present application is based on the application with CN application number 202211541435.8 and the filing date of December 01, 2022, and claims its priority. The disclosure content of the CN application is hereby incorporated into the present application in its entirety.

### Technical Field

The present application relates to a method for separating and purifying CVB1. Specifically, the present application relates to a method for purifying CVB1 using a heparin affinity medium.

### Background Art

*Enterovirus* belongs to the genus *Enterovirus* of the family *Picornaradae.* It is a kind of non-enveloped positive-sense single-stranded RNA virus with a virus particle that has a regular icosahedron structure with a diameter of about 30 nm, and comprises viral capsid and viral nucleic acid from the exterior to the interior. Enteroviruses mainly include poliovirus, echovirus, coxsackievirus and new type enteroviruses. According to the pathogenicity in suckling mice, coxsackieviruses (CV) can be divided into two groups, Group A and Group B. Group A contains 23 serotypes, and Group B contains 6 serotypes.

Coxsackievirus B1 (CVB1) is a common human pathogen, the susceptible population thereof is infants and young children, and its main clinical symptoms include common cold symptoms such as fever, headache, diarrhea, and occasionally serious diseases such as aseptic meningitis, myocarditis, hepatitis, pancreatitis and hand-foot-and-mouth disease. The mortality rate after CVB1 infection is low.

Due to the small genome of CVB1, which enables it to penetrate the blood-brain barrier, and the mild symptoms elicited in adults after infection, CVB1 has unique advantages and great potential as an oncolytic virotherapy drug. Developing CVB1 into a vaccine or therapeutic drug has high requirements on the purification and preparation processes. First of all, the purification process needs to effectively remove relevant impurities, including host proteins, host nucleic acids, and process additives, such as antifoaming agent, nuclease, etc. Moreover, the virus separation and purification process should also be robust, reproducible, and suitable for commercial scale-up production.

At present, there are relatively few reports on the separation and purification methods of coxsackievirus. In these studies, in order to obtain coxsackievirus with higher-purity, purification methods such as ultracentrifugation and density gradient centrifugation are usually used. However, such methods have problems such as small sample volume, low throughput, long operation time, and low virus purity and recovery rate.

### Contents of the present application

After extensive research, the inventors of the present application developed a method for purifying CVB1 based on a heparin affinity medium. The method has a high CVB1 infection titer recovery rate, excellent impurity removal effect, low requirements for the initial sample to be purified, simple purification process, can be used to prepare complete CVB1 particles on a large scale, and can meet the requirements for yield and purity in industrialization.

Therefore, the present application provides a method for purifying CVB1 (Coxsackievirus type B1) in a sample, which comprises a step of contacting the sample with a heparin affinity medium.

In certain embodiments, the method comprises subjecting the sample to a heparin affinity chromatography.

In certain embodiments, the heparin affinity chromatography uses a medium with heparin or a heparin mimetic as a ligand, such as Capto Heparin, UniGel^{®}-65 Heparin or Cellufine^{™} Sulfate.

In certain embodiments, the Capto Heparin includes, but is not limited to, Capto Heparin commercially available from Cytiva.

In certain embodiments, the UniGel^{®}-65 Heparin includes, but is not limited to, UniGel^{®}-65 Heparin commercially available from Suzhou Nanomicro.

In certain embodiments, the Cellufine^{™} Sulfate includes, but is not limited to, Cellufine^{™} Sulfate commercially available from JNC Corporation.

In certain embodiments, the heparin affinity chromatography comprises the following steps:
(1) in a first solution, allowing CVB1 in the sample to bind the heparin affinity chromatography medium to form a complex;
(2) in a second solution, subjecting the complex to dissociation; and,
(3) collecting a CVB1-containing dissociation product.

In some embodiments, the first solution has an electric conductance close to that of the sample, for example, the first solution has an electric conductance of 10 to 15 mS/cm; and/or the second solution has an electric conductance of 30 to 50 mS/cm, for example, the second solution contains 300 to 500 mM NaCl.

In some embodiments, the first solution and/or the second solution has a pH value close to that of the sample, for example the first solution and/or the second solution has a pH value of 6.5 to 8.0.

In certain embodiments, before step (1), the method further comprises a step of adjusting the electric conductance and/or pH value of the sample.

In some embodiments, before step (1), the method further comprises: adjusting the electric conductance of the sample to 10 to 15 mS/cm, and/or adjusting the pH value of the sample to 6.5 to 8.0.

In some embodiments, the first solution and the sample both have an electric conductance of 10 to 15 mS/cm.

In some embodiments, the first solution and the sample both have a pH value of 6.5 to 8.0.

In some embodiments, the pH value of the first solution, the pH value of the second solution and the pH value of the sample are all 6.5 to 8.0.

In some embodiments, in step (1), the heparin affinity chromatography is performed with a sample loading volume of 10 to 20 column volumes.

In certain embodiments, the heparin affinity chromatography is performed with a CVB1 retention time of 10 to 30 minutes.

Although there have been cases in the art of using heparin affinity chromatography for the purification of some viruses, due to the diversity of different viral structures, whether different viruses can bind to heparin or their heparin-binding characteristics (e.g., affinity, specificity, etc.) vary widely. For example, it has been clearly disclosed in the art that wild-type CVB3 (Coxsackievirus type B3) does not bind to heparin, and the inventors of the present application have for the first time implemented the use of heparin affinity chromatography for separation and purification of CVB1 (Coxsackievirus type B1), and achieved remarkable purification effect.

In certain embodiments, the method further comprises subjecting the sample to size exclusion chromatography, adsorption chromatography, and/or, composite chromatography.

In certain embodiments, the method further comprises subjecting the sample to size exclusion chromatography, adsorption chromatography, and/or multiplex chromatography after the heparin affinity chromatography.

In certain embodiments, the adsorption chromatography uses a chromatography medium that is capable of adsorbing HCP (host cell protein) and/or HCD (host cell DNA).

In certain embodiments, the composite chromatography uses a multimodal composite chromatography medium that combines size exclusion and anion exchange, such as Capto Core400 or Capto Core700.

In certain embodiments, the composite chromatography uses a chromatography medium that is a size exclusion chromatography medium capable of adsorbing HCP (host cell protein) and/or HCD (host cell DNA).

In certain embodiments, the composite chromatography uses a chromatography medium that is Capto Core400.

In certain embodiments, the composite chromatography comprises the following steps:
(I) passing an equilibration buffer through the composite chromatography medium;
(II) passing a CVB1-containing sample through the composite chromatography medium;
(III) passing an elution buffer through the composite chromatography medium.

In some embodiments, in step (II), the loading volume of the sample is 5 to 20 column volumes.

In certain embodiments, the composite chromatography is performed with a CVB1 retention time of 1 to 10 min.

In certain embodiments, the equilibration buffer and/or the elution buffer have an electric conductance close to the electric conductance of the sample in step (II), for example, the equilibration buffer and/or the elution buffer have an electric conductance of 10 to 20 mS/cm.

In some embodiments, before step (II), the method further comprises adjusting the sample to have an electric conductance of 10 to 20 mS/cm.

In certain embodiments, the electric conductance of the equilibration buffer and the electric conductance of the elution buffer are both 10 to 20 mS/cm.

In certain embodiments, the electric conductance of the equilibration buffer, the electric conductance of the elution buffer, and the electric conductance of the sample are all 10 to 20 mS/cm.

In certain embodiments, the equilibration buffer is the same as or similar to the elution buffer.

In some embodiments, the composite chromatography uses a chromatography column with a height of 10 to 30 cm.

In certain embodiments, the CVB1 in the sample is present in a free form (e.g., in a cell culture supernatant) or is present in a cell.

In certain embodiments, all or a portion of the CVB1 in the sample is present in a cell.

In certain embodiments, the sample is a viral culture containing host cell components, and the method further comprises subjecting the sample to a pretreatment before performing chromatography, in which the pretreatment comprises a step of lysing the cell to release CVB1.

In certain embodiments, the pretreatment comprises steps of:
(i) lysing the cell to release CVB1; and,
(ii) removing or reducing a cell debris and/or particulate matter in a cell lysate.

In certain embodiments, in step (i), the method comprises lysing the cell by using a cell lysis solution to releases CVB1.

In certain embodiments, the cell lysis solution comprises a surfactant.

In certain embodiments, the surfactant comprises Tween-20, Tween-80, or a combination thereof.

In certain embodiments, the surfactant has a concentration of 0.25% to 1% (m/v).

In certain embodiments, the cell lysis solution comprises 0.25% to 1% (m/v) Tween-20, Tween-80, or a combination thereof.

In certain embodiments, the method comprises lysing the cell by using the cell lysis solution at a temperature of 25 to 37°C for 1 to 4 hours.

In certain embodiments, during or after step (i), the pretreatment further comprises a step of removing or reducing a host cell nucleic acid.

In certain embodiments, the method comprises removing or reducing the host cell nucleic acid by using a nuclease.

In certain embodiments, the method comprises removing or reducing the host cell nucleic acid by adding the nuclease to the cell lysis solution or adding the nuclease to the cell lysate.

In certain embodiments, in the nuclease digestion system, the nuclease has a concentration of 20 to 100 U/ml.

In certain embodiments, the nuclease digestion system further comprises magnesium ions.

In some embodiments, the concentration of magnesium ions in the nuclease digestion system is 1 to 2 mM.

In some embodiments, the method comprises removing or reducing the host cell nucleic acid by (a) performing an enzymatic digestion using the nuclease for 1 to 4 hours at a temperature of 25 to 37°C, or (b) performing an enzymatic digestion using the nuclease for 4 to 24 hours at a temperature of 2 to 8°C.

In certain embodiments, in step (ii), the method comprises removing a cell debris and/or particulate matter from the cell lysate by filtration.

In certain embodiments, the filtration is single-stage filtration or multi-stage filtration.

In some embodiments, the filter used in the filtration step is a depth filter (e.g., a 0.04 to 9 µm depth filter) or a two-stage capsule filter (e.g., a 5 µm+0.65 µm two-stage capsule filter).

In certain embodiments, the pretreatment further comprises step (iii): removing or reducing a small molecule (e.g., a molecule with a molecular weight less than 300 KDa) in the product of step (ii).

In certain embodiments, in step (iii), the method comprises performing ultrafiltration and diafiltration of the product of step (ii) using an ultrafiltration system to remove or reduce a small molecule (e.g., a molecule with a molecular weight less than 300 KDa) therein, and concentrating the product.

In certain embodiments, the ultrafiltration system comprises a hollow fiber or an ultrafiltration membrane bag.

In certain embodiments, the ultrafiltration system has a molecular weight cutoff of 50 to 300 KDa (e.g., 50 KDa, 70 KDa, 100 KDa, 300 KDa).

In certain embodiments, the replacement solution used when performing ultrafiltration is sodium acetate, histidine, sodium phosphate or Tris buffer.

In certain embodiments, the replacement solution has a pH value of 6 to 9.

In some embodiments, the replacement solution has an electric conductance of 8 to 20 mS/cm.

In certain embodiments, the replacement solution comprises 80 to 200 mmol/L NaCl.

In some embodiments, the concentration is performed with a concentration factor of 2 to 10 times.

In certain embodiments, the pretreatment does not comprise step (iii): removing or reducing a small molecule (e.g., a molecule with a molecular weight less than 300 KDa) in the product of step (ii).

In certain embodiments, the method comprises directly subjecting the product of step (ii) to chromatography.

In certain embodiments, the method further comprises adjusting the electric conductance and/or pH value of the product of step (ii) before subjecting the product of step (ii) to chromatography.

### Definition of Terms

In the present application, unless otherwise stated, scientific and technical terms used herein have the meanings commonly understood by those skilled in the art. Moreover, the procedures of virology, biochemistry, and immunology laboratory used herein are routine procedures widely used in the corresponding fields. Meanwhile, in order to better understand the present application, definitions and explanations of relevant terms are provided below.

When the terms "for example," "e.g.," "such as," "comprise," "include," or variations thereof are used herein, these terms should not be considered as limiting terms and should instead be interpreted to mean "without limitation" or "not being limited to."

Unless otherwise indicated herein or clearly contradicted by context, the terms "a" and "an" as well as "the" and similar referents in the context of describing the present application (especially in the context of the following claims) are to be construed to cover singular and plural.

As used herein, the term "heparin affinity medium" refers to an affinity medium that uses a heparin molecule or heparin mimetic as a ligand for a target molecule, and is suitable for separation and purification of a target molecule that specifically bind to the heparin molecule or heparin mimetic. Usually, in order to facilitate the separation and purification of the target molecule, the heparin molecule or heparin mimic is immobilized on a solid phase carrier (or matrix). Commonly used carriers (or matrix) are agarose gel, polyacrylamide gel, porous glass beads, dextran gel, cellulose, etc.

As used herein, the term "chromatography" refers to a separation technique that employs mobile phase and stationary phase to separate one type of molecule (e.g., CVB1 particle) from other molecules (e.g., impurities) in a sample. A liquid mobile phase is a mixture containing the specific type of molecule to be separated and other molecules. The specific type of molecule to be separated and other molecules are transported by the mobile phase across or through the stationary phase (e.g., solid matrix), and the specific type of molecule to be separated is separated from other molecules in the mobile phase due to the different interaction of different molecules in the mobile phase with the stationary phase or due to the different retention times in the stationary phase of different molecules in the mobile phase.

As used herein, the term "heparin affinity chromatography" refers to a chromatography method that relies on the specific binding of a target molecule to a heparin molecule (or heparin mimetic), in which the heparin molecule (or heparin mimetic) coupled to a solid phase carrier (or matrix) serves as a ligand and can interact with a molecule (e.g., CVB1 particle) in the mobile phase (sample), that is, the heparin molecule (or heparin mimetic) has a specific affinity for the molecule to be purified. As understood in the context of the present application, the heparin affinity chromatography involves the addition of a sample containing CVB1 particles to a stationary phase containing heparin (or heparin mimetic) as a ligand.

### Beneficial effects of the present application

Compared with traditional CVB1 purification methods, the method for purifying CVB1 provided in the present application has a high CVB1 infection titer recovery rate and excellent impurity removal effect (e.g., it can remove more than 99% of HCP (host cell protein) and HCD (host cell DNA)), has low requirements for the initial sample to be purified (e.g., CVB1 particles can be directly and efficiently captured from virus harvests), has simple purification process and can be used to prepare CVB1 intact particles on a large scale, has yield and purity that meet the requirements for industrialization, and can be used in the development of antitumor drugs, diagnostic reagents or vaccines.

The embodiments of the present application will be described in detail below with reference to the drawings and examples. However, those skilled in the art will understand that the following drawings and examples are only used to illustrate the present application and do not limit the scope of protection of the present application. Various objects and advantageous aspects of the present application will become apparent to those skilled in the art from the accompanying drawings and the following detailed description of preferred embodiments.

### Brief Description of the Drawings

Fig. 1 shows the chromatograms of heparin affinity chromatography in Example 1.
Fig. 2 shows the chromatograms of heparin affinity chromatography in Example 2.
Fig. 3 shows the chromatograms of Capto Q ImpRes anion chromatography in Comparative Example 1.
Fig. 4 shows the chromatograms of Capto Q ImpRes anion chromatography in Comparative Example 2.

### Specific Models for Carrying Out the present application

The present application will now be described with reference to the following examples which are intended to illustrate but not to limit the present application.

### Example 1: Application of heparin affinity chromatography medium in purification and preparation of CVB1

### Cultivation of virus:

HEK293 CD medium (QuaCell) was used to culture HEK293 suspension cells (QuaCell). The initial cell density was (3 to 6)×10⁵ cells/mL. The cell culture conditions were: 37°C, 5% CO₂, and rotation speed of 130 rpm; After being cultured for 3 to 5 days, the cells reached a density of (4 to 6)×10⁶ cells/mL, CVB1 virus was inoculated with MOI=0.01, the virus culture conditions were: 35°C, 5% CO₂, and rotation speed of 130 rpm, and the virus was harvested 24h to 48h after the inoculation.

### Cell lysis and enzymatic digestion:

0.5% Tween-20 was added to the harvested virus culture for cell lysis to promote the sufficient release of intracellular viruses. During lysis, a nuclease was added to digest the host-derived nucleic acids that were released into the virus-containing liquid phase composition through cell lysis. Preferred nuclease could be the nuclease from Merck, Novozyme, Yeasen or Cellnuo. The effective concentration of the nuclease was 20 to 100 U/ml. The treatment conditions for the nuclease digestion could be 25°C to 37°C for 1 to 4 h, or could be digestion at 2 to 8°C for 4h to 24h. At the same time, additional 1 to 2 mM magnesium ions were added to the lysis solution to increase enzyme activity. For the nuclease digestion products of the above conditions, the HCD (host cell DNA) contents as detected in the digestion products were all reduced to within 1% of the HCD content before digestion.

### Clarification and filtration:

After the cell lysis and nuclease digestion, the virus-containing liquid phase composition usually had a high turbidity (>700 NTU). In this example, a combination of 5 µm PP material filter and 0.65 µm PES material filter (Cobetter), or a 0.04 µm to 9 µm depth filter (Cobetter) was used to remove a large amount of cell debris and particulate matter, while the turbidity was controlled within 70 NTU, and there was almost no loss of virus activity after filtration.

### Ultrafiltration and concentration:

In this example, an ultrafiltration system equipped with a 100 KDa ultrafiltration membrane bag (Merck) or 100 KDa hollow fiber (Cobetter) was used to perform concentration and diafiltration of the clarified liquid of the virus-containing liquid composition obtained above, with a concentration factor of 2 to 10 times. The replacement solution used was a pH buffer with a certain concentration of NaCl, in which the pH buffer used could be one of sodium acetate, His, PB, and Tris with a concentration of 10 to 50mM, the preferred pH range was between 6 and 9, and the solution electric conductance was between 8 to 15 mS/cm. Ultrafiltration and diafiltration can remove a large number of small molecule impurities in virus samples.

### Purification by chromatography:

In this example, a two-step chromatography of composite chromatography and heparin affinity chromatography was used to purify the virus harvest liquid after the ultrafiltration.

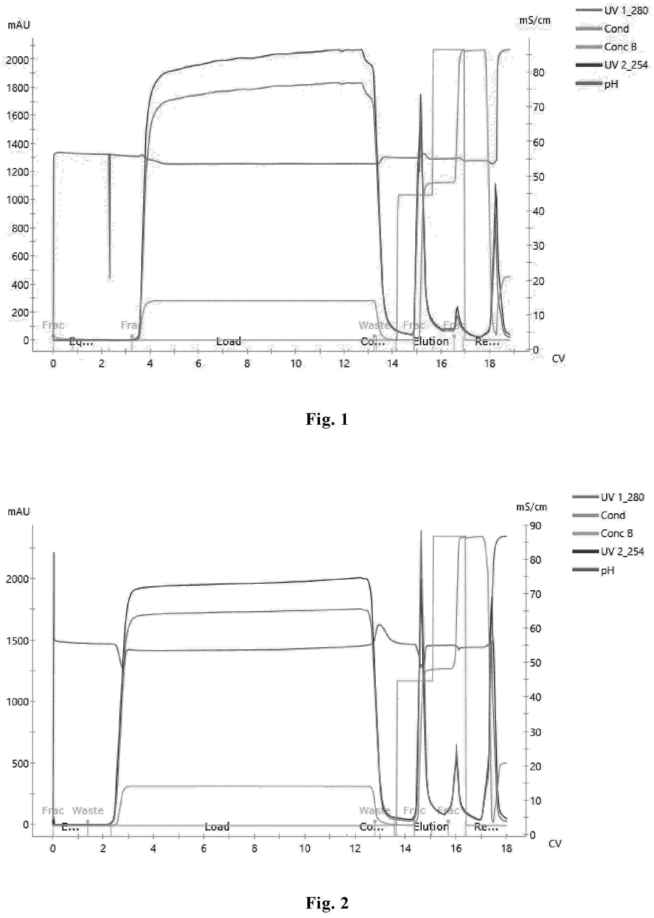

The equipment of chromatography system used for the purification was AKTA Pure (Cytiva), in which the heparin chromatography medium used was preferably Capto Heparin of Cytiva, or UniGel^{®}-65 Heparin of Nanomicro, or Cellufine^{™} Sulfate of JNC. In this example, UniGel^{®}-65 Heparin of Nanomicro was used as an example to purify the virus-containing liquid phase composition after the ultrafiltration. The chromatography column equilibrium solution had a pH value between 6.5 and 8.0, and an electric conductance of 10 to 15 mS/cm. The pH value and electric conductance of the virus-containing liquid phase composition were close to those of the equilibrium solution. The average retention time of the sample in the chromatography column was between 12 to 15 minutes, the total loading volume was 10 to 20 column volumes. A buffer containing 500 mM NaCl was used to elute the sample, the buffer had a pH value close to the pH value of the sample, and the elution peak sample was collected. In the heparin affinity chromatography of this step, a large number of impurities was in the flow-through sample, and more than 95% of HCP and HCD could be removed, while the virus was mainly in the elution peak, and the virus infection titer recovery rate reached 80% and above. The heparin affinity chromatogram was shown in Fig. 1.

The sample purified by the heparin affinity chromatography was adjusted with purified water to reach an electric conductance of 10 to 20 mS/cm, and Core400 chromatography was used to further remove smaller impurities. The loading volume of the Core400 chromatography was 5 to 20 CV, and the flow-through sample was collected.

The purification process in this example could remove more than 99% of HCP (host cell protein) and HCD, and the virus infection titer recovery rate reached 60% and above.

### Example 2: Application of heparin affinity chromatography medium to directly capture CVB1 in harvest liquid

In this example, a heparin affinity chromatography medium was used to directly capture CVB1 from a virus harvested liquid that had not underwent ultrafiltration, concentration and diafiltration.

The procedures for virus cultivation, cell lysis, enzymatic digestion, clarification and filtration were as described in Example 1.

### Purification by chromatography:

The equipment of chromatography system used for purification was AKTA Pure (Cytiva), in which the heparin chromatography medium used could be preferably Capto Heparin of Cytiva, or UniGel^{®}-65Heparin of Nanomicro, or Cellufine^{™} Sulfate of JNC. In this example, UniGel^{®}-65Heparin of Nanomicro was used as an example for direct capture of the virus-containing liquid phase composition after the clarification and filtration. The chromatography column equilibrium solution had a pH value between 7.5 and 8.0, and an electric conductance of 10 to 15 mS/cm. The pH value and electric conductance of the virus-containing liquid phase composition were close to those of the equilibrium solution. The average retention time of the sample in the chromatography column was between 10 and 30 minutes, and the total loading volume was 10 to 20 column volumes. A buffer containing 500 mM NaCl was used to elute the sample. The pH value of the buffer was close to the pH value of the sample. The elution peak sample was collected. In the heparin affinity chromatography of this step, a large number of impurities was in the flow-through sample, and more than 95% of HCP and HCD could be removed, the virus was mainly in the elution peak, and the virus infection titer recovery rate reached 80% and above. The heparin affinity chromatogram was shown in Fig. 2.

The sample purified by the heparin affinity chromatography was adjusted with purified water to reach an electric conductance of 10 to 20 mS/cm, and Core400 chromatography was used to further remove smaller impurities. The loading volume of the Core400 chromatography was 5 to 10 CV, and the flow-through sample was collected.

The purification process in this example could remove more than 99% of HCP (host cell protein) and HCD, and the virus infection titer recovery rate reached 60% and above. In this example, the heparin affinity chromatography was used to directly capture CVB1 from the virus harvested liquid without ultrafiltration, concentration and diafiltration, which reduced the process of ultrafiltration, concentration and diafiltration, significantly simplified the purification process, and improved purification efficiency.

### Comparative Example 1: Purification and preparation of CVB1 using anion exchange chromatography method

The virus cultivation, cell lysis and enzymatic digestion, clarification, filtration and ultrafiltration procedures were as described in Example 1.

### Purification by chromatography:

In this comparative example, the virus sample was purified by anion exchange chromatography, and then it was purified by chromatography with composite medium, and the flow-through peak was collected to obtain the pure virus-containing liquid.

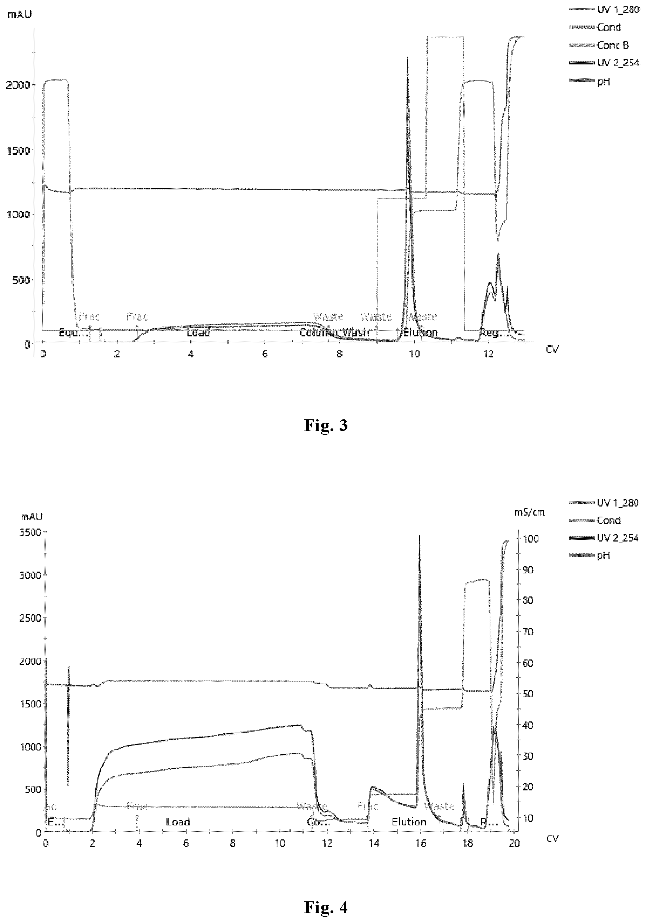

The equipment of purification chromatography system used was AKTA Pure (Cytiva), and the anion chromatography medium used was preferably Capto Q ImpRes of Cytiva. The equilibrium solution used for Capto Q ImpRes chromatography was the same as the ultrafiltration displacement solution, with a pH value of 6 to 9 and an electric conductance of 8 to 15 mS/cm. The ultrafiltered virus-containing liquid phase composition was loaded to Capto Q ImpRes chromatography, in which the average retention time of the sample was 5 to 15 minutes, and the total loading volume was 5 to 10 column volumes. After loading, an eluate buffer containing 300 to 500 mM NaCl was used for elution, the pH of the eluate buffer was consistent with the pH of the equilibrium solution, and the elution peak was collected. In the anion chromatography of this step, about 50% of the impurities were in the flow-through sample, and the virus was mainly in the elution peak. The virus infection titer recovery rate reached 70% and above. The anion exchange chromatogram was shown in Fig. 3.

The sample purified by the anion chromatography was adjusted with purified water to reach an electric conductance of 10 to 20 mS/cm, and Core400 chromatography was used to further remove smaller impurities. The loading volume of the Core400 chromatography was 5 to 10 CV, and the flow-through sample was collected.

The two-step chromatography process based on this comparative example was not as effective as the heparin affinity chromatography in removing HCP and HCD, its virus infection titer recovery rate was 40% and above, and because the Capto Q ImpRes chromatography could non-specifically bind all negatively charged proteins, its overall loading capacity is lower than that of the affinity chromatography, and the loading sample volume needs to be controlled below 10 CV, otherwise penetration would be easy to occur.

### Comparative Example 2: Application of anion exchange chromatography to directly purify CVB1 in harvest liquid

In this comparative example, an anion exchange chromatography medium was used to directly purify CVB1 from the virus harvest liquid without ultrafiltration, concentration and diafiltration.

The procedures for virus cultivation, cell lysis, enzymatic digestion, clarification and filtration were as described in Example 1.

### Purification by chromatography:

The equipment of chromatography system used for purification was AKTA Pure (Cytiva), and the anion chromatography medium used was Capto Q ImpRes of Cytiva. In the anion exchange chromatography purification experiment of virus, when the sample loading volume was 5 to 10 CV, most of the virus sample was not bound to the anion exchange resin, and the main virus products and impurities were in the flow-through peak. By adjusting the pH values and electric conductance of the equilibrium solution, loading sample and eluate solution, and prolonging the retention time of the virus in the anion exchange resin, more than 90% of the virus was still in the flow-through sample, and failed to be captured by the anion exchange resin. It was speculated that the reason could be that a large number of impurities in the matrix competitively bound to the active sites of the anion exchange resin, so that the anion exchange resin was unable to effectively capture viruses. The chromatogram was shown in Fig. 4.

Although the specific embodiments of the present application have been described in detail, those skilled in the art will understand that various modifications and changes can be made to the details based on all teachings that have been disclosed, and these changes are within the protection scope of the present application. The full scope of the present application is given by the appended claims and any equivalents thereof.

## Claims

1. A method for purifying CVB1 (Coxsackievirus type B1) in a sample, which comprises a step of contacting the sample with a heparin affinity medium.

2. The method according to claim 1, wherein the method comprises subjecting the sample to a heparin affinity chromatography;
preferably, the heparin affinity chromatography uses a medium with heparin or a heparin mimetic as a ligand, such as Capto Heparin, UniGel^{®}-65 Heparin or Cellufine^{™} Sulfate;
preferably, the heparin affinity chromatography comprises the following steps:
(1) in a first solution, allowing CVB1 in the sample to bind the heparin affinity chromatography medium to form a complex;
(2) in a second solution, subjecting the complex to dissociation; and,
(3) collecting a CVB 1-containing dissociation product;
preferably, the electric conductance of the first solution is close to the electric conductance of the sample, for example, the electric conductance of the first solution is 10 to 15 mS/cm; and/or the electric conductance of the second solution is 30 to 50 mS/cm, for example, the second solution contains 300 to 500 mM NaCl;
preferably, the pH value of the first solution and/or the second solution is close to the pH value of the sample, for example, the pH value of the first solution and/or the second solution is 6.5 to 8.0;
preferably, before step (1), the method further comprises a step of adjusting the electric conductance and/or pH value of the sample;
preferably, before step (1), the method further comprises adjusting the electric conductance of the sample to 10 to 15 mS/cm, and/or adjusting the pH value of the sample to 6.5 to 8.0;
preferably, in step (1), the heparin affinity chromatography is performed with a sample loading volume of 10 to 20 column volumes;
preferably, the heparin affinity chromatography is performed with a CVB 1 retention time of 10 to 30 minutes.

3. The method according to claim 2, wherein the method further comprises subjecting the sample to size exclusion chromatography, adsorption chromatography, and/or composite chromatography;
preferably, the method further comprises subjecting the sample to size exclusion chromatography, adsorption chromatography, and/or composite chromatography after the heparin affinity chromatography.

4. The method according to claim 3, wherein the adsorption chromatography uses a chromatography medium that is capable of adsorbing HCP (host cell protein) and/or HCD (host cell DNA);
preferably, the composite chromatography uses a multimodal composite chromatography medium that combines size exclusion and anion exchange, such as Capto Core400 or Capto Core700.

5. The method according to claim 4, wherein the composite chromatography comprises the following steps:
(I) passing an equilibration buffer through the composite chromatography medium;
(II) passing a CVB1-containing sample through the composite chromatography medium;
(III) passing an elution buffer through the composite chromatography medium;
preferably, in step (II), the loading volume of the sample is 5 to 20 column volumes;
preferably, the composite chromatography is performed with a CVB 1 retention time of 1 to 10 minutes;
preferably, the equilibration buffer and/or the elution buffer have an electric conductance close to that of the sample in step (II), for example, the equilibration buffer and/or the elution buffer have an electric conductance of 10 to 20 mS/cm;
preferably, before step (II), the method further comprises adjusting the sample to have an electric conductance of 10 to 20 mS/cm;
preferably, the equilibration buffer is the same as or similar to the elution buffer;
preferably, the composite chromatography uses a chromatography column with a column height of 10 to 30 cm.

6. The method according to any one of claims 1 to 5, wherein the sample is a viral culture containing host cell components, and the method further comprises subjecting the sample to a pretreatment before performing the chromatography, and the pretreatment comprises a step of lysing a cell to release CVB1;
preferably, the pretreatment comprises steps of:
(i) lysing the cell to release CVB1; and,
(ii) removing or reducing a cell debris and/or particulate matter in a cell lysate.

7. The method according to claim 6, wherein in step (i), the method comprises lysing the cell by using a cell lysis solution to release CVB1;
preferably, the cell lysis solution comprises a surfactant;
preferably, the surfactant comprises Tween-20, Tween-80, or a combination thereof;
preferably, the surfactant has a concentration of 0.25% to 1% (m/v);
preferably, the cell lysis solution comprises 0.25% to 1% (m/v) Tween-20, Tween-80, or a combination thereof;
preferably, the method comprises lysing the cell by using the cell lysis solution at a temperature of 25 to 37°C for 1 to 4 hours.

8. The method according to claim 6 or 7, wherein, in step (i) or after step (i), the pretreatment further comprises a step of removing or reducing a host cell nucleic acid;
preferably, the method comprises removing or reducing the host cell nucleic acid by using a nuclease;
preferably, the method comprises removing or reducing the host cell nucleic acid by adding the nuclease to the cell lysis solution or adding the nuclease to the cell lysate;
preferably, in the nuclease digestion system, the nuclease has a concentration of 20 to 100 U/ml;
preferably, the nuclease digestion system further comprises magnesium ions;
preferably, the concentration of magnesium ions in the nuclease enzymatic hydrolysis system is 1 to 2 mM;
preferably, the method comprises removing or reducing the host cell nucleic acid by (a) performing an enzymatic digestion using the nuclease at a temperature of 25 to 37°C for 1 to 4 h, or (b) performing an enzymatic digestion using the nuclease at a temperature of 2 to 8°C for 4 to 24 h.

9. The method according to any one of claims 6 to 8, wherein, in step (ii), the method comprises removing a cell debris and/or particulate matter from the cell lysate by filtration;
preferably, the filtration is single-stage filtration or multi-stage filtration;
preferably, the filter used in the filtration step is a depth filter (e.g., a 0.04 to 9 µm depth filter) or a two-stage capsule filter (e.g., a 5 µm+0.65 µm two-stage capsule filter).

10. The method according to any one of claims 6 to 9, wherein the pretreatment further comprises step (iii): removing or reducing a small molecule (e.g., a molecule with a molecular weight less than 300 KDa) in the product of step (ii);
preferably, in step (iii), the method comprises subjecting the product of step (ii) to ultrafiltration and diafiltration by using an ultrafiltration system to remove or reduce a small molecule (e.g., a molecule with a molecular weight less than 300 KDa) therein, and concentrating the product;
preferably, the ultrafiltration system comprises a hollow fiber or ultrafiltration membrane bag;
preferably, the ultrafiltration system has a molecular weight cutoff of 50 to 300 KDa (e.g., 50 KDa, 70 KDa, 100 KDa, 300 KDa);
preferably, the replacement solution used during ultrafiltration is sodium acetate, histidine, sodium phosphate or Tris buffer;
preferably, the pH value of the replacement solution is 6 to 9;
preferably, the electric conductance of the replacement solution is 8 to 20 mS/cm;
preferably, the replacement solution comprises 80 to 200 mmol/L NaCl;
preferably, the concentration is performed with a concentration factor of 2 to 10 times.

11. The method according to any one of claims 6 to 9, wherein the pretreatment does not comprise step (iii): removing or reducing a small molecule (e.g., a molecule with a molecular weight less than 300 KDa) in the product of step (ii);
preferably, the method comprises directly subjecting the product of step (ii) to chromatography;
preferably, the method further comprises adjusting the electric conductance and/or pH value of the product of step (ii) before performing chromatography on the product of step (ii).
